# EUROPEAN PATENT APPLICATION

(11) **EP 1 205 192 A1**
(43) Date of publication of application: **15.05.2002**
(21) Application number: 00870261.5
(22) Date of filing: 07.11.2000
(51) Int. Cl.: A61L 9/03, A01M 1/20

(54) **Efficient self-heating device for dispensing volatile materials**

(71) Applicant: THE PROCTER & GAMBLE COMPANY, Cincinnati, Ohio 45202 (US)
(72) Inventor: Blenkiron, Peter, Surrey, Egham TW20 8XB (GB); Burrowes, Lee, Surrey, Woking GU21 4PR (GB); Curtis, Terence Graham, Bucks, High Wycombe HP11 1JN (GB)
(74) Representative: Canonici, Jean-Jacques

(57) **Abstract**

Disclosed are devices for dispensing volatile materials such as fragrances into the atmosphere surrounding each such device. The device contains a convection zone which holds the materials to be volatilized and dispensed. The convection zone also has both an air inlet end and a convection current discharge end which are open or openable to the atmosphere. Volatilization of materials within, and discharge of these materials from, the convection zone is promoted by the generation of heat via the non-combustive oxidation reaction of thermogenic materials held within a heat generation zone in the device. Heat transferred to the convection zone from the heat generation zone creates convective flow of air and volailized active material through the convection zone and out of its discharge end into the surrounding atmosphere.

## Description

### Technical Field/Field of the Invention

The present invention relates to a self-heating device for dispensing volatile materials such as fragrances into the atmosphere surrounding the device.

### Background of the Invention

Devices for providing and dispensing volatile materials are well-known in the art. Such devices can include room freshners or fragrancing articles, vaporizers for humidification or dispensing and dispersal of therapeutic vapors, incense sticks, fragrancing or insect-repelling candles, and the like. Such devices generally involve utilization of some source of heat which promotes the volatilization of the materials to be dispensed and, of course, some source of the volatile or volatilizable materials themselves.

Some fragrancing devices, such as candles and incense sticks, involve use of an open flame or an active combustion reaction to provide the source of heat which promotes volatilization. Besides the obvious drawbacks of the hazards of using such types of heat sources, arrangements which involve flames or combustion are not especially portable and cannot be used, for example, in automobiles or around flammable materials.

Other types of dispensing devices for volatiles use electrical energy as a heat source. Plug-in room air-fresheners, for example, are commercially marketed under the tradenames *Ambi-Pur* and *Glade.* The amount of electrical energy required to operate devices of this type renders such devices relatively non-portable.

Some articles and devices for emitting or dispensing volatile materials employ non-combustive thermogenic materials as a heat source useful for promoting volatilization of the ingredients to be emitted or dispensed. Villamarin; PCT Patent Application WO 91/07996; Published June 13, 1991, for example, discloses a self-contained fragrance delivery package, comprising a fluent volatilizable fragrance material and an air-activatable chemical heat source. Disclosed configurations include a layered pad with covered perforations which can be removed to expose the heat source to air and activate the pad. Other dispensing devices using a non-combustive chemical heat source are disclosed in Takei; U.S. Patent 4,330,506; Issued May 18, 1982; Koiso et al; U.S. Patent 4,516,564; Issued May 14, 1985 and Arao; Japanese Patent Application 63175771; Published January 26, 1990.

Known dispensing devices for volatiles in general are not self-contained and readily portable or they may have problems with efficient transfer of heat to the volatilizable materials or effective discharge of the volatile materials from the device or article into the surrounding atmosphere. Thus, there is a continuing need to provide dispensing devices in forms which are safe, portable, and especially effective at discharging the desired volatile materials in suitable amounts and over an acceptably prolonged period of time.

### Summary of the Invention

The present invention is directed to a dispensing device for discharging volatile materials into the atmosphere surrounding the device. The invention further relates to the use of such a device for discharging volatiles such as fragrances. The devices herein comprise two separate zones. One zone is a convection zone which is preferably substantially rigid and which contains the volatilizable materials to be discharged from the device. The convection zone has both an air inlet end and a convection current discharge end. Both ends are open or openable to the atmosphere surrounding the device. The volatilizable active material found in the convection zone is placed there in a manner which permits, and does not interfere with, the flow of convection air currents through the convection zone.

The other essential zone in the devices herein is a heat generation zone which is in thermodynamic communication with the convection zone. The heat generation zone contains thermogenic materials which are capable of producing thermal energy via a non-combustive reaction when contacted with air. The thermal energy produced by the thermogenic materials can be transferred to the convection zone to thereby volatilize the volatilizable material therein . Such heat transfer to the convection zone also provides convective flow of air and volatilized active material through the convection zone and into the atmosphere surrounding the device.

In preferred configurations, the dispensing devices herein further comprise means for controlling the contact of air with the thermogenic materials in the heating zone. Frequently this involves providing the convection and heat generation zones of the devices herein in an insert which is held within a surrounding holder. Such a holder for this zone-containing insert can comprise both a substantially rigid base and a substantially rigid cover for the base. The base and cover of the holder, prior to activation of the device, can keep the thermogenic materials in the heat generation zone of the insert from contact with air. The device can then be activated by altering the holder to provide air openings therein. Upon activation in this manner, heat is generated in the heat generation zone which then enhances volatilization of the materials in the convection zone and promotes their emission into the surrounding atmosphere.

### Brief Description of the Drawings

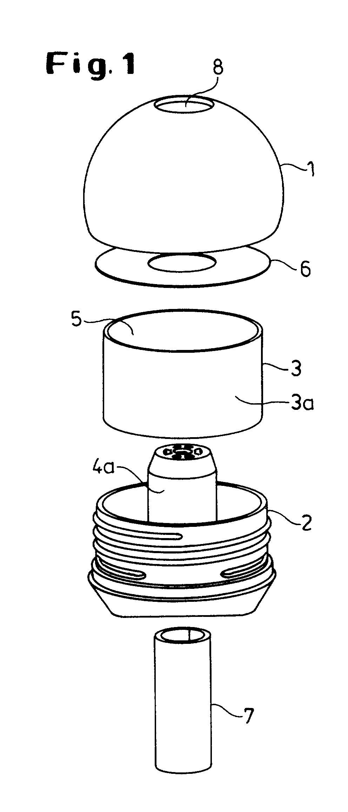

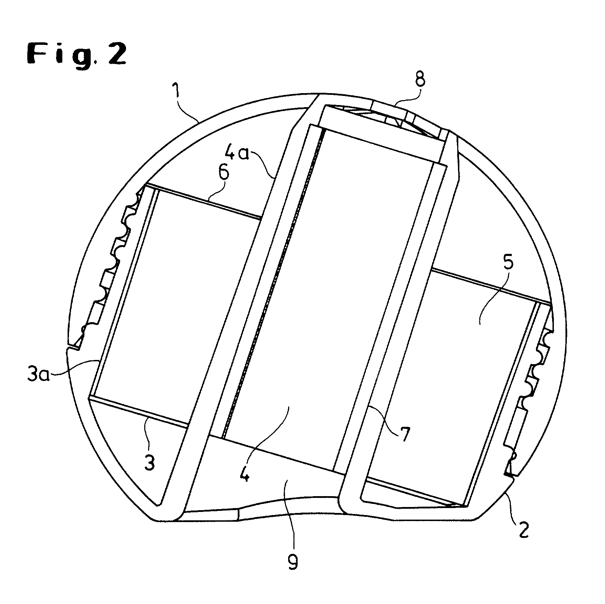

The drawings show an exploded view (Figure 1) and a section view (Figure 2) of an assembled fragrance dispensing device according to the present invention.

### Detailed Description of the Invention

The dispensing devices of the present invention comprise both convection and heat generation zones. In preferred configurations, these zones are found within an insert which is carried and held within a two-part holder. All of the elements of the dispensing devices herein are described in detail as follows with respect to such a preferred configuration :

### A) Insert

An insert is the component of the preferred dispensing devices herein which holds both the volatilizable materials to be emitted from the device and the thermogenic materials which serve to provide the thermal energy to help volatilize such materials. The thermogenic materials which are held within the heat generation zone, and preferably also the volatilizable materials which are held within the convection zone, will be sealed from the atmosphere surrounding the insert until the device is activated by exposing the contents of the zones in the insert to the outside air.

### i) Convection Zone

One essential component of the devices, and preferably of an insert which forms part of such devices, herein is the convection zone. It is the convection zone which holds the volatilizable materials which the dispensing devices of this invention are designed to emit. The convection zone can be of any shape or configuration but will generally be substantially rigid.

Preferably the convection zone will have a volume of from 1 cm³ to 50 cm³, more preferably from 3 cm³ to 15 cm³. It will also preferably be elongated, having a major dimension of from 1 cm to 15 cm, more preferably from 2 cm to 10 cm. Preferably the convection zone will be configured within the device, e.g., within the insert, such that the major dimension of the convection zone is generally in a vertical position as the insert is held within the dispensing device.

Frequently the convection zone, e.g., within the insert, will be generally cylindrical in configuration. Alternatively, the convection zone can have a tapered configuration. Of whatever configuration, the convection zone must be open or openable to the surrounding atmosphere at both of its ends.

In the cylindrical configuration, the convection zone will typically have a circular cross-sectional area which ranges from 1 cm² to 10 cm² more preferably from 2 cm² to 6 cm². In the tapered configuration, the convection zone will generally have a smaller circular cross-sectional area at or near the top of the zone than at or near the bottom of the zone. For the tapered configuration, the circular cross-sectional area at or near the top of the convection zone will typically range from 0.5 cm² to 5 cm², more preferably from 1 cm² to 3 cm², and the circular cross-sectional area at or near the bottom will typically range from 1 cm² to 10 cm², more preferably from 2 cm² to 6 cm². In the tapered configuration, the volatilizable materials tend to move through the convection zone faster at or near the top by virtue of the effect of the Bernulli principle. This helps to boost the effectiveness of the volatilized material emission into the atmosphere surrounding the device.

The convection zone, as with the rest of the elements of the dispensing devices herein, can be made of any suitable material which provides the requisite rigidity, strength, heat resistance/heat conductivity and compatibility with the materials to be held therein. Typically thermoplastic or thermosetting materials such as polyethylene, polypropylene, polystyrene, acrylic resins, and the like, may be utilized to form the convection zone, e.g., within the insert.

The convection zone will contain the volatilizable materials which are to be emitted from the device when the convection zone is exposed to the surrounding atmosphere. For purposes of this invention, the term "volatilizable materials" will include materials which may already be largely in the vapor phase at ambient temperature (e.g. 20°C). Volatilizable materials also include those which may be completely or partially in a liquid or solid phase at ambient temperature but which can be vaporized by the heat generated by the thermogenic materials in the heat generation zone of the devices herein.

Suitable volatilizable materials are those which can act as fragrances, insect repellents, insecticides and materials which are or which produce therapeutic vapors. Preferred volatilizable materials are fragrances which can include any odoriferous materials having a vapor pressure of at least 17.5 mmHg @ 20 °C. Generally, the devices herein will contain from 0.1 gram to 10 grams, more preferably from 0.5 gram to 3 grams, most preferably from 1 gram to 2 grams of the volatilizable materials within the convection zone.

The volatilizable materials may be placed into the convection chamber in neat form, or they may be held in the convection zone in combination with a liquid or solid carrier substrate. Any material which will absorb, adsorb, or otherwise hold or carry the volatilizable material can be employed as carrier or carrier substrate. The carrier material, however, must permit the flow of convective air currents through he convection zone once the device is activated and the contents of the convection zone begin to be heated.

Especially suitable carriers can include solid absorbent materials such as felt or cellulosics including paper, cotton, airfelt, and the like. Other carrier include porous polyolefin materials. An especially preferred carrier for materials to be held in the convection zone is the porous polypropylene material marketed under the tradename, Accurel™. Typically the weight ratio of carrier material to volatilizable active materials in the convection zone will range from 100:1 to 1:50, more preferably from 50:1 to 1:20.

### ii) Heat Generation Zone

The convection zone herein is juxtaposed and in thermodynamic communication with at least one heat generation zone containing air-activatable thermogenic materials. The heat generation zone, like the convection zone, can be constructed of any suitable material which will hold thermogenic materials and be compatible with such materials and the heat generated thereby when the devices herein are activated. Preferably the heat generation zone will have a volume of 10 cm³ to 100 cm³, more preferably from 20 cm³ to 40 cm³.

In preferred embodiments wherein the convection zone is generally cylindrical, the heat generation zone will be of generally annular configuration, surrounding the convection zone, with the generally cylindrical convection zone running through the core of the heat generation zone.

The heat generation zone will contain thermogenic materials which produce thermal energy via a non-combustive oxidation reaction upon contact with oxygen-containing gas such as for example upon exposure to and contact with air. Typically such materials are those used to bring about the exothermic oxidation of iron and are used in particulate form. Generally the heat generation zone will contain from 10 grams to 50 grams, more preferably from 15 grams to 30 grams, of the thermogenic material combination.

A preferred combination of air-activatable thermogenic materials comprises from 30% to 80% by weight of the combination of iron powder, from 3% to 25% by weight of the combination of carbon particles, from 0.5% to 10% by weight of the combination of a metal salt such as alkali metal or alkaline earth metal salts, and from 1% to 40% by weight of the combination of water. Such thermogenic material combinations may also contain an additional water-holding particles (e.g., vermiculite, porous silicates) present to the extent of from 0.1% to 30% by weight of the combination. Other optional ingredients in the thermogenic material combination include oxidation reaction enhancers (chromium, manganese, copper), hydrogen gas inhibitors (sodium hydroxide, sodium thiosulfate), fillers (cellulosics), anti-caking agents (tricalcium phosphate, sodium silicoaluminates), thickeners (starches) and surfactants. Thermogenic materials of the type useful in the heating zone of the inserts herein are described in greater details in Burkett et al; U.S. Patent 5,918,590; Issued July 6, 1999.

The thermogenic materials used in the heat generation zone should, upon activation, be capable of producing a temperature within the heat generation zone of at least 60 °C within a period of from 3 to 7 minutes after contact of air with the thermogenic materials is initiated. Preferably, this temperature will be maintained for a period of at least 180 minutes, more preferably at least 240 minutes, after activation of the device.

The thermogenic materials in the heat generation zone are preferably held and constrained within this zone by air-permeable holding means. Such holding means serves to prevent the generally solid, e.g., granular, thermogenic materials from being removed from the device, either purposefully or unintentionally, once the device is opened and the heat generation zone is thereby exposed to the outside atmosphere. Such air-permeable holding means, can comprise, for example, fabric- or polymer-based mesh materials having mesh openings, e.g., apertures, therein which are large enough to permit suitable flow of air therethrough but small enough to prevent significant amounts of the solid thermogenic material from passing through the mesh.

### B. Holder

The inserts of the preferred device configuration, as described hereinbefore, are held with the convection chamber in a generally upright vertical position by a holder component for the insert. In the preferred devices herein, the holder comprises a substantially rigid base and a substantially rigid cover for the base. The shape of the holder cover and holder base will, of course, depend upon the shape and configuration of the insert which is to fit inside the holder. For inserts which are generally cylindrical in configuration, the base and cover comprising the holder will generally be cylindrical or spherical.

The cover and base elements of the holder may be configured to control or alter the flow of air into and through the dispensing device. This can be accomplished by providing the cover and/or base with openable air holes which can be opened to activate the device. The cover and base elements of the holder may, for example, be rotatable or otherwise moveable in relation to each other in order to open, close or vary the size of the air inlet openings.

The holder may also preferably be constructed of thermochromic material such that at least a portion of the outside surface of the holder changes color upon heating. In this manner, the device can be constructed of a material which changes color when the device is activated and the holder heats up as a result of thermal energy generation in the heat generation zone(s). This provides a signal to the user of the device that the device has been activated and is generating heat and emitting volatiles.

A preferred dispensing device according to the present invention is one having a cylindrical insert held within a spherical holder. Such a device is depicted in Figures 1 and 2 of the drawing. In the two figures there is shown the dispensing device holder comprising a cover **1** which screws onto a base **2**. Inside the cover and base is a generally cylindrical insert **3**. The insert surrounds a generally cylindrical convection zone **4** formed into the base **2** along the axis of the insert as defined by cylinder wall **4a**. The convection zone **4** is thus surrounded by an annular chamber comprising the heat generation zone **5** as defined by the inside cylinder wall **4a** and the outside wall **3a** of the insert. Positioned within the convection zone **4** is a carrier tube **7** of Accurel™ polypropylene onto which is absorbed an effective amount of a perfume material (not shown). Positioned within the annular heat generation zone **5** is an effective amount of thermogenic materials (not shown). The thermogenic materials are held in the heat generation zone by an air permeable screen **6**. The convection zone **4** has an open upper end **8** and an open lower end **9**.

### Mode of Operation

When the cover **1** is tightly screwed onto the base **2**, the thermogenic material in the heat generation zone **5** is hermetically sealed from the outside atmosphere. To activate the device shown in the drawings, the cover **1** is partially unscrewed from the base **2**. This then allows air from the outside atmosphere to pass into the device, through the air permeable screen **6** and into contact with the thermogenic material held within the heat generation zone **5**. The resulting thermogenic reaction in the heat generation zone releases thermal energy which is transferred through wall **4a** into the convection zone **4**. This thermal energy helps volatilize the perfume material adsorbed onto the carrier **7** of Accurel™ material. The thermal energy so transferred also creates convection air currents through the convection zone **4** from top to bottom and thus promotes discharge of the volatilized perfume into the atmosphere surrounding the device.

Other preferred configurations for dispensing devices of the type disclosed herein involve inserts with rupturable sealing means and/or inserts with multiple heating chambers. Devices of these types are described in greater detail in the concurrently filed European Patent Application No._________ (P&G Case No. CM-2468F) and European Patent Application No.__________ (P&G Case No. CM-2469F).

## Claims

1. A device suitable for providing improved discharge of volatilized active materials into the atmosphere surrounding such a device, said device being **characterized in that** it comprises:
A) a convection zone having an air inlet end and a convection current discharge end, both ends being open or openable to the atmosphere ;
B) a volatilizable active material placed within said convection zone in a manner which permits the flow of convective air currents through said convection zone; and
C) a heat generation zone in thermodynamic communication with said convection zone, said heat generation zone containing thermogenic materials capable of producing thermal energy via a non-combustive reaction when contacted with air, said thermogenic materials further being capable of transferring thermal energy so produced to said convection zone to thereby volatilize said volatilizable active material and to further thereby provide convective flow of air and volatilized active material through said convection zone and out of the discharge end of said convection zone into the surrounding atmosphere.

2. A device suitable for providing improved discharge of volatilized active materials into the atmosphere surrounding such a device, said device being **characterized in that** it comprises:
A) a convection zone having an air inlet end and a convection current discharge end, both ends being open or openable to the atmosphere ;
B) a carrier substrate material positioned within said convection zone in a manner which permits the flow of convective air currents through said convection zone, said carrier substrate material being loaded with a volatilizable active material; and
C) a heat generation zone in thermodynamic communication with said carrier substrate material, said heat generation zone containing thermogenic materials capable of producing thermal energy via a non-combustive reaction in contact with air, said thermogenic materials further being capable of transferring thermal energy so produced to said carrier substrate material to thereby volatilize said volatilizable active material and to further thereby provide convective flow of air and volatilized active material through said convection zone and out of the discharge end of said convection zone into the surrrounding atmosphere.

3. A device according to Claim 2 wherein said carrier substrate material is selected from the group consisting of paper, cotton, felt, airfelt, and porous polyolefins.

4. A device according to Claim 2 or Claim 3 wherein the weight ratio of carrier material to volatilizable active material ranges from 100:1 to 1:50.

5. A device according to any of Claims 1 to 4 wherein the volatizable active material is selected from the group consisting of fragrances, insect repellents, insecticides and materials producing therapeutic vapors.

6. A device according to any of Claims 1 to 5 wherein the thermogenic materials are capable of generating thermal energy via an iron oxidation reaction.

7. A device according to Claim 6 wherein said thermogenic materials comprise a combination of iron, carbon, water and inorganic salts.

8. A device according to any of Claims 1 to 7 wherein said thermogenic materials are capable of producing a temperature within said heat generation zone of at least 60 °C within a period of from 3 to 7 minutes after contact of air with said thermogenic materials is initiated.

9. A device according to any of Claims 1 to 8 which further comprises means for controlling the contact of air with said thermogenic materials.

10. A device according to Claim 9 wherein said means for controlling the contact of air with said thermogenic materials is provided by fashioning said convection zone and said heat generating zone into an insert which is placed within a holder, said holder having openings therein which are open or openable to the atmosphere surrounding said device.

11. A device according to Claim 10 wherein said holder is constructed of thermochromic material such that at least a portion of the outside surface of said holder changes color when said device is activated and thermal energy is produced therein.

12. A device according to any of Claims 1 to 11 wherein said convection zone has a generally vertical major dimension which ranges in length from 1 cm to 15 cm.

13. A device according to Claim 12 wherein said convection zone is generally cylindrical.

14. A device according to Claim 12 wherein said convection zone is generally of tapered configuration such that its horizontal cross-sectional area at or near the top of its vertical dimension is smaller than its horizontal cross-sectional area at or near the bottom of its vertical dimension.

15. A device according to any of Claims 1 to 14 wherein said convection zone has a horizontal cross-sectional area which is generally circular.

16. A device according to Claim 15 wherein said heat generation zone is of generally annular configuration and completely surrounds said convection zone.

17. Use of an active material discharge device according to any of Claims 1 to 16 by initiating contact of said thermogenic materials within said device with air.
